# EUROPEAN PATENT SPECIFICATION

(11) **EP 2 742 519 B1**
(45) Date of publication and mention of the grant of the patent: **16.12.2015**
(21) Application number: 12751648.2
(22) Date of filing: 01.08.2012
(51) Int. Cl.: H01G 9/12

(54) **SINTERED CAPACITOR ELECTRODE INCLUDING A 3-DIMENSIONAL FRAMEWORK**
SINTERKONDENSATORELEKTRODE MIT EINEM DREIDIMENSIONALEN RAHMEN
ELECTRODE DE CONDENSATEUR FRITTÉE COMPRENANT UNE ARCHITECTURE TRIDIMENSIONNELLE

(30) Priority: 11.08.2011 US 201161522541 P
(43) Date of publication of application: 18.06.2014
(73) Proprietor: Cardiac Pacemakers, Inc., St. Paul, MN 55112-5798 (US)
(72) Inventor: SHERWOOD, Gregory, J., North Oaks, MN 55127 (US)
(74) Representative: Peterreins Schley
(86) International application number: PCT/US2012/049132
(87) International publication number: WO 2013/022666

(56) References cited:
- WO-A1-01/57928
- WO-A2-2011/075506
- US-A1- 2010 246 100

## Description

### TECHNICAL FIELD

This document relates generally to energy storage and particularly to sintered electrodes including a 3-Dimensional ("3D") framework.

### BACKGROUND

Electrical stimulation therapy can benefit some patients. For example, some patients suffer from an irregular heartbeat or arrhythmia and may benefit from application of electrical stimulation to the heart. Some patients suffer from a particular type of arrhythmia known as fibrillation. Fibrillation may affect different regions of the heart, such as the atria or the ventricles. When a fibrillation occurs in the ventricles, the heart's ability to pump blood is dramatically reduced, putting the patient at risk of harm. It has been found that applying an electrical stimulation to the patient can effectively treat patients suffering disorders such as from fibrillation by restoring a regular heartbeat.

Because disorders such as fibrillation can happen at any time, it is helpful to have a device that is easily accessible. In some cases, it is helpful if that device is portable or implantable. In developing a device that is portable or implantable, lightweight and compact components are desirable.

The following are background documents:

US Patent 5,728,490, serial no. 08/671,527, references a three-dimensional substrate material for use in constructing battery electrodes comprises a sintered matrix material selected from the group consisting of reticulated metal foams, conductive fibers and metal powder compacts, and a porous covering layer of a polymeric mesh material, flexible metal screen or metal fibers, bonded to at least one surface of the matrix material to retain the sintered matrix material substantially within the planar surface of the surface of matrix material during spiral-wounding of the chemically loaded matrix material. The document does not reference capacitors.

US Patent Publication No. 2007/0248887, serial no. 11/408,856, references a high-performance lithium-based battery using metal foam (or some other three-dimensional, fillable, conductive material) to make its electrodes. The document does not reference capacitors.

US Patent Publication No. 2010/0259866, serial no. 12/297,811, references an energy storage device includes a supercapacitor having first and second electrodes, each including a composite of a mat of conducting fibers bound by an electrolytic resin.

International Patent Publication No. WO 2011/075506 A2 discloses a capacitor case sealed to retain electrolyte, at least one anode disposed in the capacitor case, the at least one anode comprising a sintered portion disposed on a substrate, an anode conductor coupled to the substrate in electrical communication with the sintered portion, the anode conductor sealingly extending through the capacitor case to an anode terminal disposed on the exterior of the capacitor case with the anode terminal in electrical communication with the sintered portion, a cathode disposed in the capacitor case, a separator disposed between the cathode and the anode and a cathode terminal disposed on an exterior of the capacitor case and in electrical communication with the cathode, with the anode terminal and the cathode terminal electrically isolated from one another.

International Patent Publication No. WO 2001/057928 A1 discloses anode and/or cathode of a capacitor having a large surface area.

U.S. Patent Publication No. 2010/0246100 A1 discloses a solid electrolytic capacitor includes at least one capacitor element in which the other end of an anode lead extends beyond an exposed portion of an electrolyte layer exposed from a cathode layer.

### SUMMARY

The present invention relates to an apparatus as set out in claim 1, a method as set out in claim 9 and a system as set out in claim 13. Further embodiments are described in the dependent claims.

Sintered aluminum powder can be used to produce a capacitor electrode that has greater surface area and higher capacitance than other approaches, including some etched electrodes. Sintered electrodes offer improved surface area, even after oxidation, and thus offer improved energy density. These electrodes may be used to produce capacitors that are lighter and more compact.

In some examples, electrodes such as anodes are produced by printing a powder and binder mix onto an aluminum sheet. In some examples, printing is aligned on both sides of the sheet. In certain instances, the sheet serves as a base for printing. In some examples, the sheet serves as a current collector for the electrode. A finished electrode is produced once the powder material is sintered, and the sintered material is oxidized.

Such a planar electrode, however, can suffer from warping and curvature, caused, at least in part, by certain manufacturing processes, including, but not limited to, heating, pressing, coining and the like. This sheet approach is also limited to producing electrodes of a certain thickness, as printing can build up a limited amount of material onto a sheet.

In an example, the present subject matter can provide a solution to the problem of warping and curvature of a planar electrode, such as by providing an electrode including a sintered portion disposed on a current collector formed of a 3D framework defining cells extending to three axes such as orthogonal axes, the 3D framework formed into a desired shape that resists warpage or curvature.

### BRIEF DESCRIPTION OF THE DRAWINGS

The drawings illustrate generally, by way of example, various embodiments discussed in the present document. The drawings are for illustrative purposes only and may not be to scale.
FIG. 1 is a schematic of a medical system including a sintered capacitor, according to some embodiments.
FIG. 2 is an implanted medical system including a sintered capacitor, according to some embodiments.
FIG. 3A is a perspective view of a sintered 3D framework defining open areas disposed along three axes, according to various examples.
FIG. 3B is a close-up view of a sintered 3D framework defining open areas disposed along three axes such as the close-up view 3B depicted in FIG. 3A.
FIG. 4 is a plan view of an expanded metal 3D framework, according to some examples.
FIG. 5 is a schematic illustrating a process to cast an electrode, according to some examples.
FIG. 6A is a plan view of a 3D framework defining open areas disposed along three axes, according to various examples.
FIG. 6B is a cross section taken along line 4B -- 4B in FIG. 4A, showing contour.
FIG. 7A is a plan view of a capacitor including a slug electrode, according to various embodiments.
FIG. 7B is a cross section taken along line 7B -- 7B in FIG. 7A
FIG. 8A is a plan view of a capacitor, according to various examples.
FIG. 8B is a cross section taken along the line 8B -- 8B in FIG. 8A, showing a stack of electrodes formed of a 3D framework.
FIG. 9 is a method of making a capacitor, according to some embodiments.

### DETAILED DESCRIPTION

This document relates to sintered electrodes for use in an electrical energy storage device. Specific examples include sintered anodes formed of aluminum or its alloys. Certain examples are for use in aluminum electrolytic capacitors. Examples include electrodes with a sintered portion disposed onto a current collector formed of a 3D framework, such as a framework defining cells extending to three axes.

Some examples include a stack of electrodes in which at least one electrode includes a current collector formed of a 3D framework, wherein the electrodes are interconnected. In some examples, a capacitor using a single sintered electrode is provided, with a part count of three (3) including a cathodic case, an anode slug electrode disposed in the case, and separator between them. The present systems and methods improve upon energy storage devices that do not use the 3D framework at least because the electrode may be formed without undesirable warpage.

Sintering results in many interstices (e.g., spaces) between grains of the electrode. Sintered electrodes, in part, resemble crushed grains with interstices between the grains. In various examples, the interstices are filled with electrolyte, thereby increasing capacitance per unit of volume, as capacitance is proportional to a surface area exposed to electrolyte. An electrode with such interstices offers improved lateral or parallel movement of electrons in relation to a major surface of a flat electrode layer, as etched electrodes restrict lateral movement because the etchings result in voids that are typically perpendicular to the major surface of the flat layer. Accordingly, some examples have a lower ESR (equivalent series resistance) compared to etched framework defining cells extending to three axes due to this enhanced ionic flow. In various examples, the cells range from around 10 to 15 micrometer in average diameter. In some examples, one or more particulates of powdered material disposed in a cell have an average diameter of around three to four micrometers. In various examples, an oxide of around 0.5 micrometers in thickness is formed on a framework.

Overall, an energy storage device using electrodes including sintered material disposed on a 3D framework is well suited for use in an implantable medical device such as a defibrillator. Because these electrodes can produce a variety of shapes, they can be used to create energy storage devices such as capacitors that have custom shapes. The interstices are very small, making the electrodes rigid and able to withstand handling by a machine or assembly personnel. These electrodes demonstrate an improved energy density over etched electrodes and are useful to make smaller implantable devices that are able to deliver an amount of energy for a particular therapy. 3D frameworks contemplated include, but are not limited to, foam, expanded metal, fabrics, thatches, braids, scaffolding, skeleton, fins, tendrils and the like. Examples of framework include, but are not limited to, lattice in which cells are defined by conductive lattice members. Lattice forms are not limited to orthonormal lattices, as other configurations are possible. Various examples are metallic. The 3D framework examples disclosed here include features that can be used in combination, that is, aspects from one 3D framework are combinable aspects from another 3D framework, in various examples.

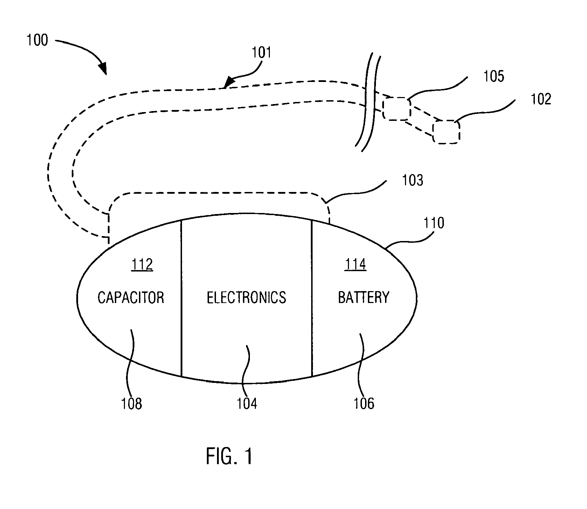

FIG. 1 is a schematic of a medical system 100 including a sintered capacitor, according to some embodiments. The medical system 100 can represent a number of systems to provide therapeutic stimulus, such as to a heart. Examples of medical systems include, but are not limited to, implantable pacemakers, implantable defibrillators, implantable nerve stimulation devices and devices that provide stimulation from outside the body, including, but not limited to, external defibrillators.

Electronics 104 can monitor the patient, such as by monitoring a sensor 105. Electronics 104 can monitor or control activity within the system 100. In some examples, the electronics 104 can monitor a patient, diagnose a condition to be treated such as an arrhythmia, and control delivery of a stimulation pulse of energy to the patient. The electronics 104 can be powered wirelessly using an inductor. Alternatively, the electronics 104 can be powered by a battery 106. In some examples, electronics 104 can direct small therapeutic bursts of energy to a patient from the battery 106.

For therapies, such as defibrillation, that use energy discharge rates exceeding what battery 106 is able to provide, a capacitor 108 is used. Energy from the battery 106 is controlled by the electronics 104 to charge the capacitor 108. The capacitor 108 is controlled by the electronics 104 to discharge to a patient to treat the patient. In some examples, the capacitor 108 entirely discharges to a patient, and in an example, the capacitor is switched on to provide therapeutic energy and switched off to truncate therapy delivery.

Some examples of a medical system 100 include an optional lead system 101. In certain instances, after implantation, the lead system 101 or a portion of the lead system 101 is in electrical communication with tissue to be stimulated. For example, some configurations of lead system 101 contact tissue with a stimulation electrode 102. The lead system 101 couples to other portions of the system 100 via a connection in a header 103. Examples of the system 101 use different numbers of stimulation electrodes and/or sensors in accordance with the needs of the therapy to be performed.

In an example, some medical systems 100 can function without a lead 101. Leadless electrode examples can be positioned in contact with the tissue to be stimulated, or can be positioned proximal to tissue to shock the tissue to be stimulated through intermediary tissue. Leadless electrodes can be easier to implant and can be less expensive as they do not require the additional lead components. The housing 110 can be used as an electrode in leadless configurations.

In certain embodiments, the electronics 104 include an electronic cardiac rhythm management circuit coupled to the battery 106 and the capacitor 108 to discharge the capacitor 108 to provide a therapeutic defibrillation pulse. In some examples, the system 100 includes an anode and a cathode sized to deliver a defibrillation pulse of at least approximately 50 joules. Other configurations can deliver larger amounts of energy. Some configurations deliver less energy. In some examples, the energy level is predetermined to achieve a delivered energy level mandated by a governing body or standard associated with a geographic region, such as a European country. In an embodiment, the anode and cathode are sized to deliver a defibrillation pulse of at least approximately 60 joules. In some examples, this is the energy level is predetermined to achieve an energy level mandated by a governing body of another region, such as the United States. In some examples, electronics 104 can control discharge of a defibrillation pulse so that the medical system 100 delivers only the energy mandated by the region in which the system 100 is used. In some examples, a pulse of 36 joules is delivered.

Packaging anodes and cathodes can reduce their efficiency. Interconnections between conductors coupled to electronics and to the electrodes of the capacitor 108 decrease efficiency, for example. Accordingly, anodes and cathodes are sized to compensate for decreases in efficiency. As such, in some embodiments, the capacitor 108 includes anodes and cathodes sized and packaged to deliver a defibrillation pulse of at least approximately 50 joules. Some are sized and packaged to deliver a defibrillation pulse of at least approximately 60 joules.

One characteristic of some sintered electrode examples is that at least one anode and cathode have a DC capacitance that is approximately 23 percent greater than a AC capacitance for the at least one anode and the cathode of an etched capacitor that has 74.5 microfarads per cubic centimeter. In some examples, the at least one anode and cathode have an AC capacitance of at least 96.7 microfarads per cubic centimeter at 445 total voltage. In some examples, this is comparable to an operating voltage of about 415 volts. This is a 30 percent improvement over an etched capacitor that has 74.5 microfarads per cubic centimeter. Total voltage is the voltage that allows 1 milliamp of leakage per square centimeter for an electrode. Some examples are aged to 415 volts.

In certain examples, the capacitor 108 includes a capacitor case 112 sealed to retain electrolyte. In some examples, the capacitor case 112 is welded. In some instances, the capacitor case 112 is hermetically sealed. In an example, the capacitor case 112 is sealed to retain electrolyte, but is sealed with a seal to allow flow of other matter, such as gaseous diatomic hydrogen or a helium molecule. Some of these examples use an epoxy seal.

A hermetically sealed device housing 110 is used to house components, such as the battery 106, the electronics 104, and the capacitor 108. Hermeticity is provided by welding components into the hermetically sealed device housing 110, in some examples. Other examples bond portions of the housing 110 together with an adhesive such as a resin based adhesive such as epoxy. Accordingly, some examples of the housing 110 include an epoxy sealed seam or port. Several materials can be used to form housing 110, including, but not limited to, titanium, stainless steel, nickel, a polymeric material, or combinations of these materials. In various examples, the housing 110 and the case 112 are biocompatible.

The capacitor 108 is improved by the present electrode technology in part because it can be made smaller and with less expense. The improvement provided by these electrodes is pertinent to any application where high-energy, high-voltage, or space-efficient capacitors are desirable, including, but not limited to, capacitors used for photographic flash equipment. The present subject matter extends to energy storage devices that benefit from high surface area sintered electrodes including, but not limited to, aluminum. The electrodes described here can be incorporated into cylindrical capacitors that are wound, in addition to stacked capacitors.

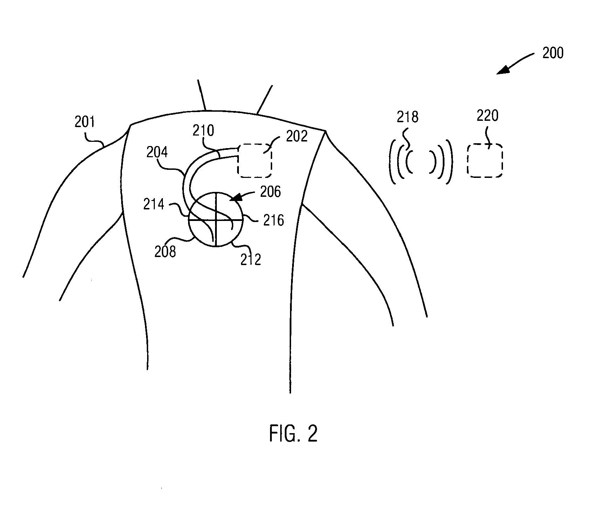

FIG. 2 is an implanted medical system 200, implanted in a patient 201, and including a sintered capacitor, according to some embodiments. The system includes a cardiac rhythm management device 202 coupled to a first lead 204 to extend through the heart 206 to the right ventricle 208 to stimulate at least the right ventricle 208. The system also includes a second lead 210 to extend through the heart 206 to the left ventricle 212. In various embodiments, one or both of the first lead 204 and the second lead 210 include electrodes to sense intrinsic heart signals and to stimulate the heart. The first lead 204 is in direct contact (e.g., touching) with the right atrium 214 and the right ventricle 208 to sense and/or stimulate both tissue regions. The second lead 210 is in direct contact with the left atrium 216 and the left ventricle 212 to sense and/or stimulate both tissue regions. The cardiac rhythm management device 202 uses the lead electrodes to deliver energy to the heart, either between electrodes on the leads or between one or more lead electrodes and the cardiac rhythm management device 202. In some examples, the cardiac rhythm management device 202 is programmable and wirelessly communicates 218 programming information with a programmer 220. In some examples, the programmer 220 wirelessly 218 charges an energy storage device of the cardiac rhythm management device 202.

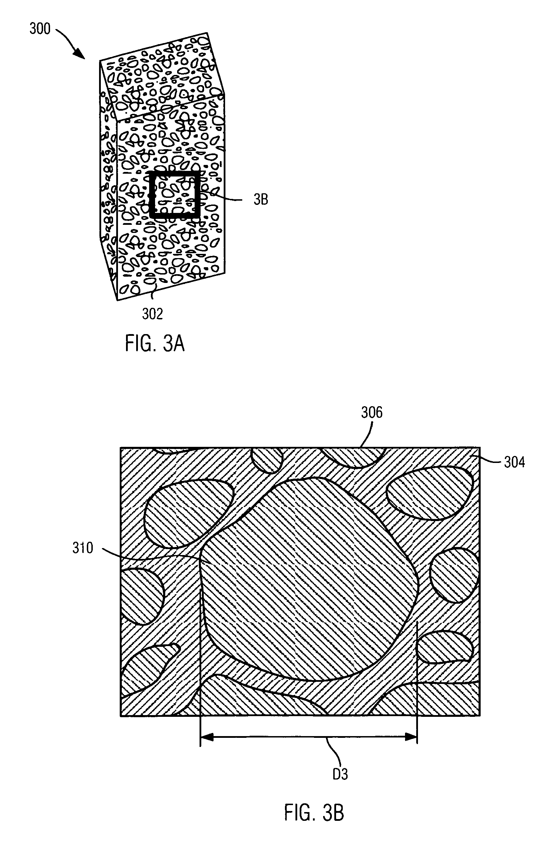

FIG. 3A is a perspective view of a 3D framework 300, according to various examples. FIG. 3B is a close-up view of a 3D framework such as the close-up view 3B depicted in FIG. 3A. The 3D framework pictured is porous and cathodic, but the present subject matter is not so limited. The 3D framework 300 is metallic in certain examples. As used herein, metallic materials are of, or relate to, being a metal, containing a metal or having properties of a metal. Metallic materials can be formed of aluminum, titanium, stainless steel, other metals or combinations of those metals. In certain examples, the 3D framework 300 is continuous. A continuous 3D framework 300 demonstrates a regular distribution of grain boundaries, as opposed to a discontinuous metal with material between grain boundaries, such as welded metals.

In some instances, the 3D framework is formed of foam. Some examples include metallic foams. In various examples, the 3D framework 300 is formed of metallic foam defining porous cells. In Figure 3B, a cell 310 is defined by the framework 304. In an example, a cell 310 is a volume that has a larger diameter D3 than a pore 306, which is a passageway extending between cells. The cell 310, according to various examples, represents a bubble in the foam. At least some of the cells are open. Pores 306 are filled with sintered material, in various examples.

In various examples, the 3D framework 300 is pliable. For example, the 3D framework 300 can be compressed elastically, in certain examples. Some examples include a 3D framework compressed inelastically. As used herein, compressed relates to cells of foam pressed together and reduced in size or volume, such as by pressure. Compressed additionally means that the 3D framework 300 is flattened as though subjected to compression. In certain examples, a 3D framework 300 including a sintered material occupies 97 percent of a selected volume, with 3 percent of the selected volume occupied by interstices, such as cells or pores.

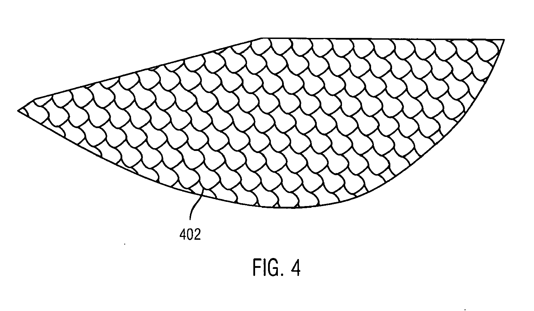

FIG. 4 is a plan view of an expanded metal 3D framework, according to some examples. The framework 402 is formed of nonexpanded metal including, but not limited to, sheet, billet, bar stock and the like. In various examples, the nonexpanded metal is formed into expanded metal by a process. Examples of processes include, but are not limited to, pressing, stretching and excising, but the present subject matter is not so limited. In some examples, a stack of frameworks can be assembled prior to sintering.

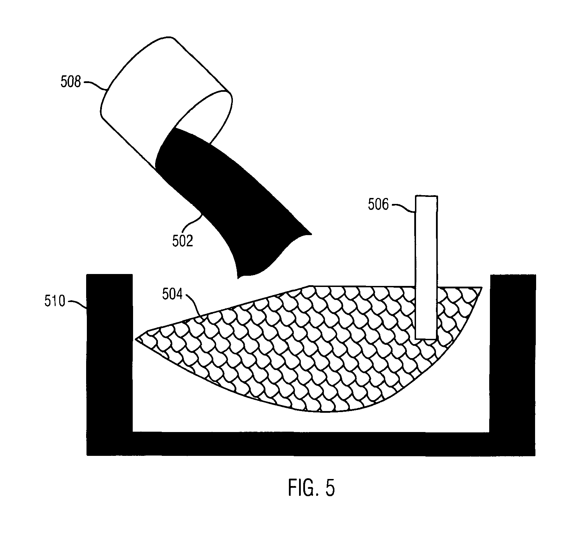

FIG. 5 is a schematic illustrating a process to cast an electrode, according to some examples. In the process, a crucible 508 pours powdered material 502 into a form 510. In various examples, powdered material 502 is cast onto a framework 504. In some examples, a feedthrough conductor 506 is cast onto the framework 504.

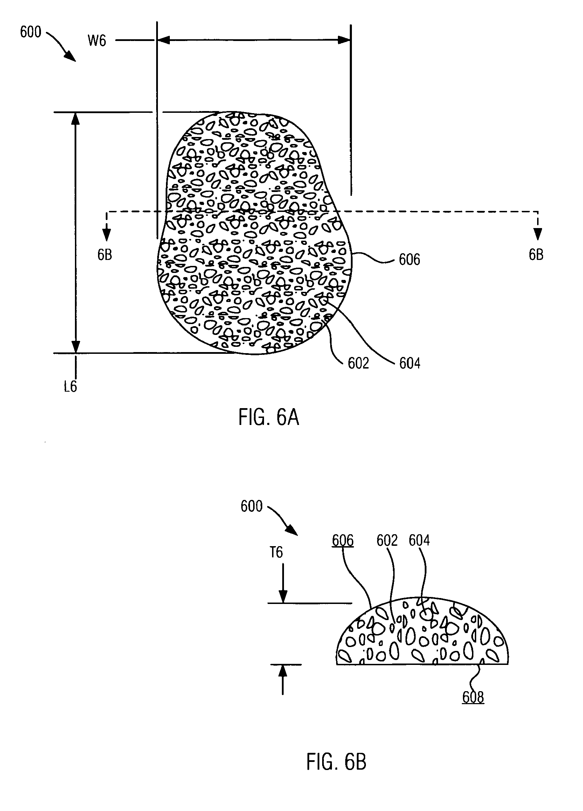

FIG. 6A is a plan view of a 3D framework 602, according to various examples. FIG. 6B is a cross section taken along line 6B -- 6B in FIG. 6A. The electrode 600 is filled with powdered material and sintered in these examples. The electrode defines a plurality of pores 604. In certain examples, the electrode has an electrode shape 606 that is selected to at least partially mate a capacitor case shape. In some instances, the electrode 600 is molded or cast into shape. In some examples, the electrode 600 is cut, such as by routing or another cutting operation. In some examples, the electrode has a flat surface 608. In certain examples, additional electrodes are stacked onto the flat surface 608. An electrode stack includes a number of electrodes, each including at least one major face that faces a major face of another electrode. In some instances, a plurality of electrodes are disposed in a stack and interconnected with one another. Interconnection can be via a conductive interconnect, for example, a weld busbar, rivet, metal spray and the like.

In various examples, the width W6 and the thickness T6 are selected such that the electrode 600 conforms to or mates with a selected capacitor case shape. Examples include a 3D framework 602 that is connected to a conductor. In certain examples, the electrode 600 is coupled to a conductor. A conductor is welded to the 3D framework 602 such that it is electrically and physically coupled to the 3D framework 602, in certain examples. In various examples, a device housing has a form factor, and the capacitor case is shaped to at least partially conform to the form factor.

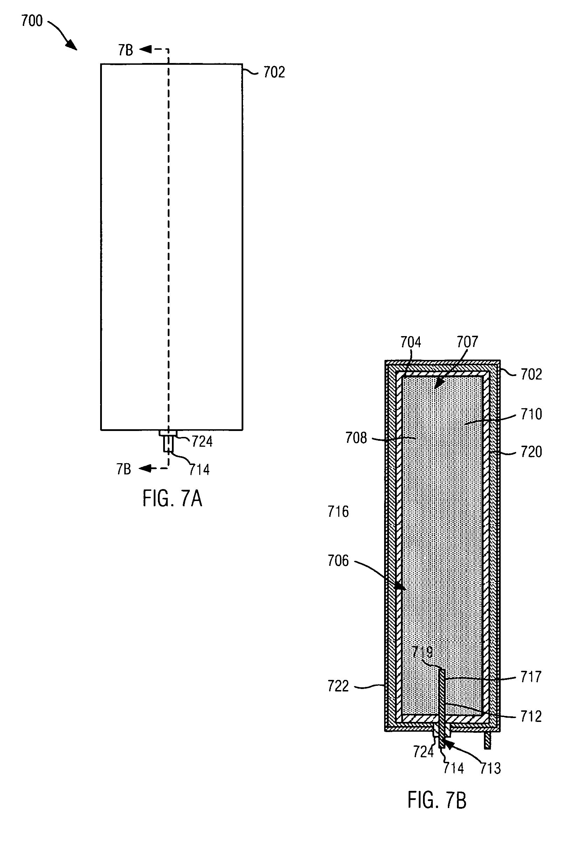

FIG. 7A is a plan view of a capacitor 700 including a slug electrode 707, according to various embodiments. FIG. 7B is a cross section taken along line 7B-7B in FIG. 7A. The anode 706 is disposed in a capacitor case 702. The anode 706 includes a sintered portion 708 disposed on a 3D framework 710. In some examples, the sintered portion 708 is sintered onto the 3D framework 710. In these examples, grains of the sintered portion 708 are mechanically and electrically coupled to the 3D framework 710 using the sintering process that forms the sintered portion 708. In some examples, the sintered portion 708 is coupled with the 3D framework 710 such as through fasteners, adhesion or welding, or combinations thereof. As used herein, fasteners can include rivets, clamps, screws, combinations of these fasteners and other fasteners to mechanically fasten components to one another.

A slug electrode 707 includes a sintered portion 708 of an anode 706 and any 3D framework 710 to which the sintered portion 708 is mechanically fixed. Some examples include a slug electrode 707, such as a standalone slug electrode, including a monolithic sintered portion 708. In some examples, a slug electrode 707 is standalone in that it is the only slug electrode of a polarity in a capacitor. A sintered portion 708 is monolithic in that it is a solid structure having a regular crystalline or grain structure with no movable subcomponents. An electrode such as anode 706 includes a slug electrode 707 plus any other portions in electrical communication with the slug electrode 707, including, but not limited to, interconnects and/or conductors such as anode conductor 714. For instance, the anode conductor 714 forms some portion of the anode 706, albeit contributing a relatively small amount of capacitance when compared with the slug electrode 707.

An anode conductor 714 is coupled with the sintered portion 708 and the 3D framework 710. The anode conductor 714 sealingly extends through the capacitor case 702 to an anode terminal 713 disposed on the exterior of the capacitor case 702, with the anode terminal 713 in electrical communication with the sintered portion 708. Some examples use a feedthrough 724. In some examples, the feedthrough includes glass. In some embodiments, the feedthrough includes epoxy. In some examples, an internal length 717 of the anode conductor 714 is disposed in the sintered portion 708 of the anode 706, with the sintered portion 708 enveloping the internal length 717. In some of these embodiments, the anode 706 is sintered to and around the internal length 717, including being sintered to an end 719 of the internal length 717 and to a side 712 of the internal length 717.

In some examples, the slug electrode is a polyhedron and the anode conductor extends through a face of the anode 706, substantially perpendicular to a face of the anode 706. Some examples include an anode 706 having a polyhedron shape with rounded edges. Anodes 706 having a curved surface or a curvilinear surface are also contemplated.

In an embodiment, the anode conductor 714 is affixed to the side of a slug electrode. These embodiments do not include the internal length 717. In some of these embodiments, an end face 719 of the anode conductor is coupled to the slug electrode. The end face 719 can be sintered or affixed another way, such as by welding, adhesion or fasteners, or combinations thereof.

Alternative examples include an anode conductor 714 coupled to the 3D framework 710, with the anode conductor 714 in electrical communication with the sintered portion 708 without being in mechanical contact with the sintered portion 708. Various coupling methods are used to join the anode conductor 714 to the 3D framework 710 including, but not limited to, welding, adhesion, fasteners, and combinations thereof.

Several materials can be used to form case 702, including, but not limited to, aluminum, titanium, stainless steel, nickel, a polymeric material, or combinations of these materials. The case 702 is sealed to retain electrolyte 704. Various electrolytes can be used including, but not limited to, Suzuki-Techno Corporation electrolyte model 1184. The case 702 includes a seal, such as a resin based seal including but not limited to epoxy, in some examples. Some examples include a rubber seal to seal case portions to one another, or to seal subcomponents such as a feedthrough to one or more case portion. In some examples, case 702 is welded together from subcomponents. In certain examples, the case 702 is hermetically sealed. Some examples include a case that includes one or more backfill ports, but the present subject matter is not so limited.

A cathode 716 is also disposed in the capacitor case 702. In some examples, the cathode 716 is a flat layer. Some of these examples are approximately 20 micrometers in thickness. In some examples, the case 702 is cathodic and is part or all of the cathode 716. In an embodiment, the case 702 is not cathodic, and the cathode is electrically isolated from the case or the case is not conductive. Material of the cathode 716 can be disposed onto the case using a coating process or another process including, but not limited to, sintering, or it can be disposed against the case without being mechanically coupled to the case.

In various embodiments a separator 720 is disposed between the cathode 716 and the sintered portion 708 and 3D framework 710 of the anode 706. The separator 720 comprises one or more layers of Kraft paper in certain examples. In various examples, a hole is cut in the separator 720 to provide a port for the anode conductor 714. The separator 720 and cathode 716 are illustrated as enveloping the slug electrode 707 (that is, bending or formed around the slug electrode 707), but in some embodiments they comprise flat layers that support the slug electrode 707 on its sides. Some examples include a cathode terminal 722 disposed on an exterior of the capacitor case 702 and in electrical communication with the cathode 716, with the anode terminal 713 of the anode conductor 714 and the cathode terminal 722 electrically isolated from one another.

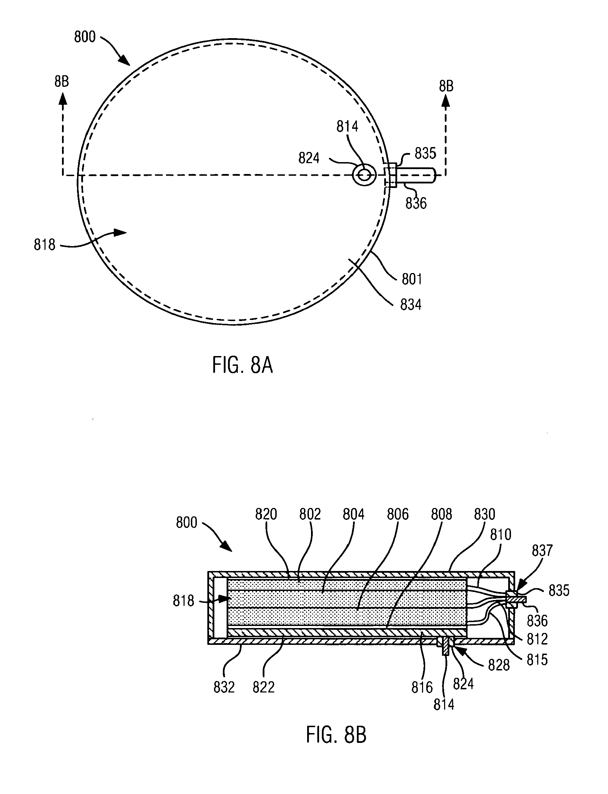

FIG. 8A is a plan view of a capacitor, according to various examples. FIG. 8B is a cross section taken along the line 8B -- 8B in FIG. 8A. Various examples include a capacitor stack 818 disposed in a capacitor case 801. The capacitor case 801, in various examples, includes a dish shaped portion 830 and a lid 832, with the lid sealed to the dish shaped portion 830, but the present subject matter is not so limited.

In various examples, the capacitor stack 818 includes a plurality of electrodes and separator. For example, a first separator 820 is disposed between the case 801 and a first electrode including a 3D framework 802 to physically separate the electrode including a 3D framework 802 from the case 801. In certain examples, the electrode including a 3D framework 802 is coupled to a conductor 810. Some instances include a second electrode including a 3D framework 804 coupled to a conductor 812. Some examples include a third electrode including a 3D framework 806 coupled to a conductor 815. In certain examples, the first, second and third electrodes abut and are in electrical communication with one another. In an example, the first, second and third electrodes abut the capacitor case 801. In some examples, the first, second and third electrodes are anodic. In various examples, the stack 818 is a stack of sintered electrodes, each adapted to stack into the stack. In certain examples, electrodes in a stack are sintered after stacking.

In certain examples, the first, second and third electrodes are electrically coupled via an interconnection between the first conductor 810, the second conductor 812 and the third conductor 815. Interconnection between the first conductor 810, the second conductor 812 and the third conductor 815 is via a conductive interconnect. Each of the conductors is electrically coupled to a respective 3D framework via welding and the like. In some instances, a conductor is formed of a metallic spray. Certain examples include a metallic ribbon coupled to the 3D framework.

In various examples, a feedthrough 837 including an electrical insulator 835 and a terminal 836 is disposed through the dish shaped portion 830 and placed into connection with the first conductor 810, the second conductor 812 and the third conductor 815 such as by welding.

Various examples include a further electrode 816. In various examples, one or more separators 808 separate the further electrode 816 from additional electrodes, such as the electrode including conductor 815. In an example, a separator 822 separates the further electrode 816 from the case 801 such as by separating the further electrode 816 from the lid 832. The further electrode 816 is cathodic, in various examples.

In various examples, the first, second and third electrodes are stacked into the dish shaped portion 830. In some examples, they are molded or cast into the dish shaped portion 830. Separator is stacked onto the third electrode, and a further electrode 816 is stacked into the dish shaped portion 830. In various examples, a lid 832 is fixed to the dish shaped portion, with a feedthrough 825 including an electrical insulator 824 and a terminal 814 is disposed through the lid 832 and placed into connection with the further electrode 816 such as by welding. In some examples, the further electrode 816 is welded to the feedthrough prior to fastening the lid 832 to the dish shaped portion 830.

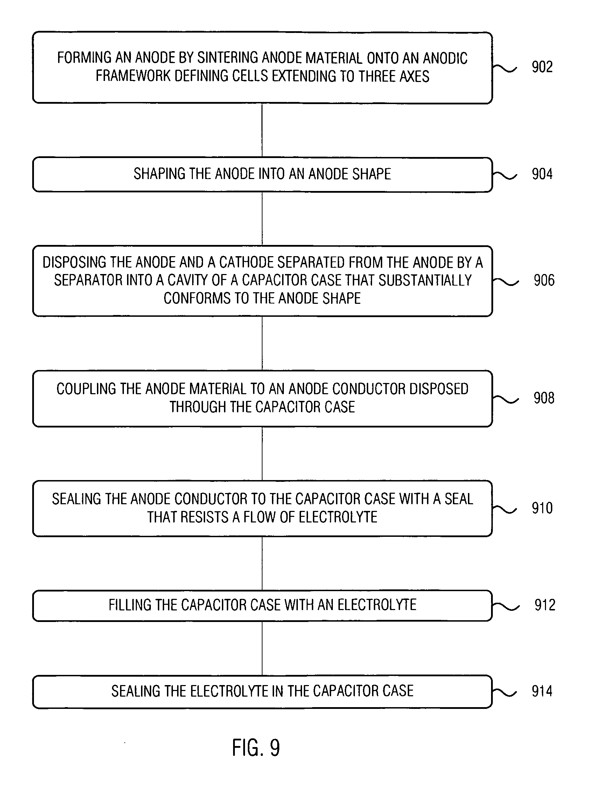

FIG. 9 is a method according to some embodiments. At 902, the method includes forming an anode by sintering anode material onto an anodic framework defining interstices such as cells or pores extending to three axes. At 904, the method includes shaping the anode into an anode shape. At 906, the method includes disposing the anode and a cathode separated from the anode by a separator into a cavity of a capacitor case that substantially conforms to the anode shape. At 908, the method includes coupling the anode material to an anode conductor disposed through the capacitor case. At 910, the method includes sealing the anode conductor to the capacitor case with a seal that resists a flow of electrolyte. At 912, the method includes filling the capacitor case with an electrolyte. At 914, the method includes sealing the electrolyte in the capacitor case.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In the event of inconsistent usages between this document and documents so incorporated by reference, the usage in this document controls.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) may be used in combination with each other. Other embodiments can be used, such as by one of ordinary skill in the art upon reviewing the above description. Also, in the above Detailed Description, various features may be grouped together to streamline the disclosure. This should not be interpreted as intending that an unclaimed disclosed feature is essential to any claim. Rather, inventive subject matter may lie in less than all features of a particular disclosed embodiment.

## Claims

1. An apparatus, comprising:
a capacitor case (112; 702; 801) sealed to retain electrolyte;
at least one electrode (600; 707) disposed in the capacitor case (112; 702; 801), the at least one electrode (600; 707) comprising a sintered portion (708) disposed on a current collector formed of a framework defining cells extending to three axes (300; 402; 504; 602; 710; 802, 804, 806);
a conductor (714; 810, 812, 815) coupled to the current collector (300; 402; 504; 602; 710; 802, 804, 806) in electrical communication with the sintered portion (708), the conductor (714; 810, 812, 815) sealingly extending through the capacitor case (112; 702; 801) to a terminal (713; 836) disposed on an exterior of the capacitor case (112; 702; 801) with the terminal (713; 836) in electrical communication with the sintered portion (708);
a second electrode (716; 816) disposed in the capacitor case (112; 702; 801);
a separator (720; 820; 808) disposed between the electrode (600; 707) and the second electrode (716; 816); and a second terminal (722; 814) disposed on the exterior of the capacitor case (112; 702; 801) and in electrical communication with the second electrode (716; 816), wherein the terminal (713; 836) and the second terminal (722; 814) electrically are isolated from one another,
**characterized in that**
the framework defining cells extending to three axes (300; 402; 504; 602; 710; 802, 804, 806) is formed of metallic foam or expanded metal.

2. The apparatus of claim 1, wherein the sintered portion (708) is disposed in the cells.

3. The apparatus of any of the preceding claims, wherein the framework (300; 402; 504; 602; 710; 802, 804, 806) is non- sintered.

4. The apparatus of any of the preceding claims, wherein the cells range from around 10 to 15 micrometer in average diameter.

5. The apparatus of any of the preceding claims, wherein the at least one electrode (600; 707) comprises a standalone slug electrode (707) that includes the sintered portion (708), with the sintered portion (708) being monolithic.

6. The apparatus of claim 5, wherein the conductor is disposed at least partially in the slug electrode (707), with the slug electrode (707) enveloping a conductive portion of the conductor.

7. The apparatus of claim 5, wherein the conductor (714; 810, 812, 815) is disposed between the slug electrode (707) and the current collector formed of a framework defining cells extending to three axes.

8. The apparatus of claim 1, wherein the at least one electrode (600; 707) is an anode that is part of a stack (818) of substantially flat electrodes including a plurality of anodes.

9. A method, comprising:
forming an anode (600; 707) by sintering anode material onto an anodic framework defining cells extending to three axes (300; 402; 504; 602; 710; 802, 804, 806);
disposing the anode (600; 707) and a cathode (716; 816) separated from the anode (600; 707) by a separator (720; 820; 808) into a cavity of a capacitor case (112; 702; 801) that substantially conforms to an anode shape;
coupling the anode material to an anode conductor (714; 810, 812, 815) disposed through the capacitor case (112; 702; 801);
sealing the anode conductor (714; 810, 812, 815) to the capacitor case (112; 702; 801) with a seal that resists a flow of electrolyte;
filling the capacitor case (112; 702; 801) with an electrolyte; and
sealing the electrolyte in the capacitor case (112; 702; 801),
**characterized in that** forming the anode includes forming the anodic framework (300; 402; 504; 602; 710; 802, 804, 806) of metallic foam or expanded metal.

10. The method of claim 9, wherein forming includes shaping the anode (600; 707) into an anode shape, preferably wherein shaping includes molding at a pressure above ambient pressure.

11. The method of any of claim 9-10, wherein forming includes printing powdered material onto the anodic framework.

12. The method of any of claim 9-11, comprising excising a plurality of anode layers from the sintering on the anodic framework defining cells extending to three axes (300; 402; 504; 602; 710; 802, 804, 806).

13. A system, comprising:
a hermetically sealed device housing (110);
a battery (106) disposed in the hermetically sealed device housing (110);
a capacitor (108) disposed in the hermetically sealed device housing (110),
the capacitor (108) comprising:
a capacitor case (112; 702; 801) sealed to retain electrolyte;
at least one anode (600; 707) disposed in the capacitor case (112; 702; 801), the at least one anode (600; 707) comprising a sintered portion (708) disposed on a current collector formed of a framework defining cells extending to three axes (300; 402; 504; 602; 710; 802, 804, 806);
an anode conductor (714; 810, 812, 815) coupled to the current collector formed of a framework defining cells extending to three axes (300; 402; 504; 602; 710; 802, 804, 806) in electrical communication with the sintered portion (708), the anode conductor (714; 810, 812, 815) sealingly extending through the capacitor case (112; 702; 801) to an anode terminal (713; 836) disposed on exterior of the capacitor case (112; 702; 801) with the anode terminal (713; 836) in electrical communication with the sintered portion (708);
a cathode (716; 816) disposed in the capacitor case (112; 702; 801);
a separator (720; 820; 808) disposed between the cathode (716; 816) and the anode (600; 707); and a cathode terminal (722; 814) disposed on the exterior of the capacitor case (112; 702; 801) and in electrical communication with the cathode (716; 816), with the anode terminal (713; 836) and the cathode terminal (722; 814) electrically isolated from one another, and an electronic cardiac rhythm management circuit (104) coupled to the battery (106) and the capacitor (108) and adapted to discharge the capacitor (108) to provide a therapeutic pulse,
**characterized in that**
the framework defining cells extending to three axes (300; 402; 504; 602; 710; 802, 804, 806) is formed of metallic foam or expanded metal.

14. The system of claim 13, wherein the capacitor (108) is sized to discharge a single therapeutically effective defibrillator pulse.

15. The system of claim 14, wherein the anode (600; 707) and the cathode (716; 816) are sized to deliver a defibrillation pulse of approximately 36 joules or wherein the anode (600; 707) and the cathode (716; 816) are sized and packaged to deliver a defibrillation pulse of approximately 36 joules.

## Patentansprüche

1. Vorrichtung, umfassend:
ein Kondensator-Gehäuse (112; 702; 801), das abgedichtet ist, um Elektrolyt zu halten;
zumindest eine Elektrode (600; 707), die in dem Kondensator-Gehäuse (112; 702; 801) angeordnet ist, wobei die zumindest eine Elektrode (600; 707) einen gesinterten Bereich (708) umfasst, der auf einem Stromsammler angeordnet ist, gebildet aus einer Struktur, die sich in drei Achsen erstreckende Zellen definiert (300; 402; 504; 602; 710; 802, 804, 806);
einen Leiter (714, 810, 812, 815), der mit dem Stromsammler (300; 402; 504; 602; 710; 802, 804, 806) verbunden ist, in elektrischer Verbindung mit dem gesinterten Bereich (708), wobei der Leiter (714; 810, 812, 815) sich abgedichtet durch das Kondensator-Gehäuse (112; 702; 801) erstreckt zu einem Anschluss (713; 836), der außen auf dem Kondensator-Gehäuse (112; 702; 801) angeordnet ist in elektrischer Verbindung mit dem gesinterten Bereich (708);
eine zweite Elektrode (716; 816), die in dem Kondensator-Gehäuse (112; 702; 801) angeordnet ist;
einen Separator (720; 820; 808), der zwischen der Elektrode (600; 707) und der zweiten Elektrode (716; 816) angeordnet ist; und
einen zweiten Anschluss (722; 814), angeordnet außen auf dem Kondensator-Gehäuse (112; 702; 801) und in elektrischer Verbindung mit der zweiten Elektrode (716; 816), wobei der Anschluss (713; 836) und der zweite Anschluss (722; 814) voneinander elektrisch isoliert sind;
**gekennzeichnet dadurch, dass**:
die Struktur, die sich in drei Achsen erstreckende Zellen definiert (300; 402; 504; 602; 710; 802, 804, 806) aus einem Metall-Schaum oder Streckmetall gebildet ist.

2. Vorrichtung gemäß Anspruch 1, wobei der gesinterte Bereich (708) innerhalb der Zellen angeordnet ist.

3. Vorrichtung gemäß irgendeinem der vorangehenden Ansprüche, wobei die Struktur (300; 402; 504; 602; 710; 802, 804, 806) nicht gesintert ist.

4. Vorrichtung gemäß irgendeinem der vorangehenden Ansprüche, wobei die Zellen einen durchschnittlichen Durchmesser in einem Bereich von 10 µm bis 15 µm haben.

5. Vorrichtung gemäß irgendeinem der vorangehenden Ansprüche, wobei die zumindest eine Elektrode (600; 707) eine alleinstehende Slug-Elektrode (707) umfasst, die den gesinterten Bereich (708) beinhaltet, wobei der gesinterte Bereich (708) monolithisch ist.

6. Vorrichtung gemäß Anspruch 5, wobei der Leiter zumindest teilweise in der Slug-Elektrode (707) angeordnet ist, wobei die Slug-Elektrode (707) einen leitfähigen Teil des Leiters umschließt.

7. Vorrichtung gemäß Anspruch 5, wobei der Leiter (714; 810, 812, 815) zwischen der Slug-Elektrode (707) und dem Stromsammler, der aus einer Struktur gebildet ist, die sich in drei Achsen erstreckende Zellen definiert.

8. Vorrichtung gemäß Anspruch 1, wobei die zumindest eine Elektrode (600; 707) eine Anode ist, die Teil eines Stapels (818) von im Wesentlichen flachen Elektroden ist, der eine Mehrzahl von Anoden beinhaltet.

9. Verfahren, umfassend:
Bilden einer Anode (600; 707) durch Sintern von Anodenmaterial auf eine Anodenstruktur, die sich in drei Achsen erstreckende Zellen definiert (300; 402; 504; 602; 710; 802, 804, 806);
Anordnen der Anode (600; 707) und einer Kathode (716; 816) getrennt von der Anode (600; 707) durch einen Separator (720; 820; 808) in einer Aussparung eines Kondensator-Gehäuses (112; 702; 801), das im Wesentlichen einer Anodenform entspricht;
Koppeln des Anodenmaterials an einen Anodenleiter (714; 810, 812, 815), der durch das Kondensator-Gehäuse (112; 702; 801) geführt ist;
Abdichten des Anodenleiters (714; 810, 812, 815) an das Kondensator-Gehäuse (112; 702; 801) mit einer Dichtung, die den Fluss von Elektrolyt verhindert;
füllen des Kondensator-Gehäuses (112; 702; 801) mit einem Elektrolyt; und
Einschließen des Elektrolyts indem Kondensator-Gehäuse (112; 702; 801), **gekennzeichnet dadurch, dass** bilden der Anode bilden der Anoden-Struktur (300; 402; 504; 602; 710; 802, 804, 806) aus einem Metall-Schaum oder Streckmetall umfasst.

10. Verfahren gemäß Anspruch 9, wobei Bilden Formen der Anode (600; 707) in eine Anodenform, bevorzugt wobei Formen Gießen bei einem Druck über Atmosphärendruck beinhaltet.

11. Verfahren gemäß irgendeinem der Ansprüche 9-10, wobei Bilden Drucken von Pulvermaterial auf die Anodenstruktur umfasst.

12. Verfahren gemäß irgendeinem der Ansprüche 9-11, weiter umfassend Ausnehmen einer Mehrzahl von Anodenschichten von dem Sintern auf die Anodenstruktur, die sich in drei Achsen erstreckende Zellen definiert (300; 402; 504; 602; 710; 802, 804, 806) umfasst.

13. System, wobei das System umfasst:
ein hermetisch abgedichtetes Vorrichtungsgehäuse (110):
eine Batterie (106), die in dem hermetisch abgedichteten Vorrichtungsgehäuse (110) angeordnet ist;
ein Kondensator (108), der in dem hermetisch abgedichteten Vorrichtungsgehäuse (110) angeordnet ist,
wobei der Kondensator (108) umfasst:
ein Kondensator-Gehäuse (112; 702; 801), das abgedichtet ist, um Elektrolyt zu halten;
zumindest eine Elektrode (600; 707), die in dem Kondensator-Gehäuse (112; 702; 801) angeordnet ist, wobei die zumindest eine Elektrode (600; 707) einen gesinterten Bereich (708) umfasst, der auf einem Stromsammler angeordnet ist gebildet aus einer Struktur, die sich in drei Achsen erstreckende Zellen definiert (300; 402; 504; 602; 710; 802, 804, 806);
einen Leiter (714, 810, 812, 815), der mit dem Stromsammler (300; 402; 504; 602; 710; 802, 804, 806) verbunden ist, in elektrischer Verbindung mit dem gesinterten Bereich (708), wobei der Leiter (714; 810, 812, 815) sich abgedichtet erstreckt durch das Kondensator-Gehäuse (112; 702; 801) zu einem Anschluss (713; 836), der außen auf dem Kondensator-Gehäuse (112; 702; 801) angeordnet ist in elektrischer Verbindung mit dem gesinterten Bereich (708);
eine zweite Elektrode (716; 816), die in dem Kondensator-Gehäuse (112; 702; 801) angeordnet ist;
einen Separator (720; 820; 808), der zwischen der Elektrode (600; 707) und der zweiten Elektrode (716; 816) angeordnet ist; und
einen zweiten Anschluss (722; 814) angeordnet außen auf dem Kondensator-Gehäuse (112; 702; 801) und in elektrischer Verbindung mit der zweiten Elektrode (716; 816), wobei der Anschluss (713; 836) und der zweite Anschluss (722; 814) voneinander elektrisch isoliert sind;
**gekennzeichnet dadurch, dass**:
die Struktur, die sich in drei Achsen erstreckende Zellen definiert (300; 402; 504; 602; 710; 802, 804, 806) aus einem Metall-Schaum oder Streckmetall gebildet ist.

14. System gemäß Anspruch 13, wobei der Kondensator (108) bemessen ist, um einen einzelnen therapeutisch aktiven Defibrillations-Impuls zu entladen.

15. System gemäß Anspruch 14, wobei die Anode (600, 707) und die Kathode (716; 816) bemessen sind, um einen Defibrillations-Impuls von ungefähr 36 Joules zu liefern oder wobei die Anode (600, 707) und die Kathode (716; 816) bemessen und verpackt sind, um einen Defibrillations-Impuls von ungefähr 36 Joules zu liefern.

## Revendications

1. Appareil, comprenant :
une enveloppe de condensateur (112 ; 702 ; 801) scellée pour retenir l'électrolyte ;
au moins une électrode (600 ; 707) disposée dans l'enveloppe du condensateur (112 ; 702 ; 801), l'au moins une électrode (600 ; 707) comprenant une partie frittée (708) disposée sur un collecteur de courant formé d'une structure définissant des cellules s'étendant sur trois axes (300 ; 402 ; 504 ; 602 ; 710 ; 802, 804, 806) ;
un conducteur (714 ; 810, 812, 815) couplé au collecteur de courant (300 ; 402 ; 504 ; 602 ; 710 ; 802, 804, 806) en communication électrique avec la partie frittée (708),
le conducteur (714 ; 810, 812, 815) s'étendant de manière étanche à travers l'enveloppe du condensateur (112 ; 702 ; 801) jusqu'à une borne (713 ; 836) disposée sur un extérieur de l'enveloppe du condensateur (112 ; 702 ; 801) avec la borne (713 ; 836) en communication électrique avec la partie frittée (708) ;
une seconde électrode (716 ; 816) disposée dans l'enveloppe du condensateur (112 ; 702 ; 801) ;
un séparateur (720 ; 820 ; 808) disposé entre l'électrode (600 ; 707) et la seconde électrode (716 ; 816) ; et une seconde borne (722 ; 814) disposée sur l'extérieur de l'enveloppe du condensateur (112 ; 702 ; 801) et en communication électrique avec la seconde électrode (716 ; 816), où la borne (713 ; 836) et la seconde borne (722 ; 814) sont électriquement isolées l'une de l'autre, **caractérisé en ce que**
la structure définissant les cellules s'étendant sur trois axes (300 ; 402 ; 504 ; 602 ; 710 ; 802, 804, 806) est formée de mousse métallique ou de métal expansé.

2. Appareil selon la revendication 1, dans lequel la partie frittée (708) est disposée dans les cellules.

3. Appareil selon l'une quelconque des revendications précédentes, dans lequel la structure (300 ; 402 ; 504 ; 602 ; 710 ; 802, 804, 806) est non frittée.

4. Appareil selon l'une quelconque des revendications précédentes, dans lequel les cellules ont un diamètre allant d'environ 10 à 15 micromètres en moyenne.

5. Appareil selon l'une quelconque des revendications précédentes, dans lequel l'au moins une électrode (600 ; 707) comprend une électrode pastille autonome (707) qui comprend la partie frittée (708), avec la partie frittée (708) étant monolithique.

6. Appareil selon la revendication 5, dans lequel le conducteur est disposé au moins partiellement dans l'électrode pastille (707), avec l'électrode pastille (707) enveloppant une partie conductrice du conducteur.

7. Appareil selon la revendication 5, dans lequel le conducteur (714 ; 810, 812, 815) est disposé entre l'électrode pastille (707) et le collecteur de courant formé d'une structure définissant des cellules étendant sur trois axes.

8. Appareil selon la revendication 1, dans lequel l'au moins une électrode (600 ; 707) est une anode qui fait partie d'un empilement (818) d'électrodes sensiblement planes comprenant une pluralité d'anodes.

9. Procédé comprenant les étapes suivantes :
former une anode (600 ; 707) en frittant un matériau d'anode sur une structure anodique définissant des cellules s'étendant sur trois axes (300 ; 402 ; 504 ; 602 ; 710 ; 802, 804, 806) ;
disposer l'anode (600 ; 707) et une cathode (716 ; 816) séparée de l'anode (600 ; 707) par un séparateur (720 ; 820 ; 808) dans une cavité d'une enveloppe de condensateur (112 ; 702 ; 801) qui se conforme sensiblement à la forme d'une anode ;
coupler le matériau de l'anode à un conducteur d'anode (714 ; 810 ; 812 ; 815) disposé à travers l'enveloppe du condensateur (112 ; 702 ; 801) ;
sceller le conducteur d'anode (714 ; 810 ; 812 ; 815) à l'enveloppe du condensateur (112 ; 702 ; 801) avec un joint qui résiste à l'écoulement d'électrolyte ; remplir d'électrolyte l'enveloppe du condensateur (112 ; 702 ; 801) ; et
sceller d'électrolyte dans l'enveloppe du condensateur (112 ; 702 ; 801),
**caractérisé en ce que** la formation de l'anode comprend de former la structure anodique (300 ; 402 ; 504 ; 602 ; 710 ; 802, 804, 806) avec de la mousse métallique ou du métal expansé.

10. Procédé selon la revendication 9, dans lequel la formation comprend la mise en forme de l'anode (600 ; 707) dans une forme d'anode, de préférence dans lequel la formation comprend d'effectuer un moulage à une pression supérieure à la pression ambiante.

11. Procédé selon l'une quelconque des revendications 9 et 10, dans lequel la formation comprend d'imprimer du métal fritté sur la structure anodique.

12. Procédé selon l'une quelconque des revendications 9 à 11, comprenant d'exciser une pluralité de couches d'anode du frittage sur la structure anodique définissant des cellules s'étendant sur trois axes (300 ; 402 ; 504 ; 602 ; 710 ; 802, 804, 806).

13. Système comprenant :
un boitier de dispositif scellé hermétiquement (110) ;
une batterie (106) disposée dans le boitier de dispositif scellé hermétiquement (110) ;
un condensateur (108) disposé dans le boitier de dispositif scellé hermétiquement (110),
le condensateur (108) comprenant :
une enveloppe de condensateur (112 ; 702 ; 801) scellée pour retenir l'électrolyte ;
au moins une électrode (600 ; 707) disposée dans l'enveloppe du condensateur (112 ; 702 ; 801), l'au moins une anode (600 ; 707) comprenant une partie frittée (708) disposée sur un collecteur de courant formé d'une structure définissant des cellules s'étendant sur trois axes (300 ; 402 ; 504 ; 602 ; 710 ; 802, 804, 806) ;
un conducteur d'anode (714 ; 810, 812, 815) couplé au collecteur de courant formé d'une structure définissant des cellules s'étendant sur trois axes (300 ; 402 ; 504 ; 602 ; 710 ; 802, 804, 806) en communication électrique avec la partie frittée (708), le conducteur d'anode (714 ; 810, 812, 815) s'étendant de manière étanche à travers l'enveloppe du condensateur (112 ; 702 ; 801) jusqu'à une borne d'anode (713 ; 836) disposée sur un extérieur de l'enveloppe du condensateur (112 ; 702 ; 801) avec la borne d'anode (713 ; 836) en communication électrique avec la partie frittée (708) ;
une cathode (716 ; 816) disposée dans l'enveloppe du condensateur (112 ; 702 ; 801) ;
un séparateur (720 ; 820 ; 808) disposé entre la cathode (716 ; 816) et l'anode (600 ; 707) ; et une borne de cathode (722 ; 814) disposée sur l'extérieur de l'enveloppe du condensateur (112 ; 702 ; 801) et en communication électrique avec la cathode (716 ; 816), avec la borne d'anode (713 ; 836) et la borne de cathode (722 ; 814) électriquement isolées l'une de l'autre, et un circuit électronique de gestion du rythme cardiaque (104) couplé à la batterie (106) et au condensateur (108) et conçu pour décharger le condensateur (108) pour délivrer une impulsion thérapeutique,
**caractérisé en ce que**
la structure définissant les cellules s'étendant sur trois axes (300 ; 402 ; 504 ; 602 ; 710 ; 802, 804, 806) est formée de mousse métallique ou de métal expansé.

14. Système selon la revendication 13, dans lequel le condensateur (108) est dimensionné pour décharger une seule impulsion de défibrillateur efficace sur le plan thérapeutique.

15. Système selon la revendication 14, dans lequel l'anode (600 ; 707) et la cathode (716 ; 816) sont dimensionnées pour délivrer une impulsion de défibrillation d'approximativement 36 joules, ou dans lequel l'anode (600 ; 707) et la cathode (716 ; 816) sont dimensionnées et agencées pour délivrer une impulsion de défibrillation d'approximativement 36 joules.
